(19) Europäisches Patentamt

European Patent Office

Office européen des brevets

(11) Publication number: **0 498 452 A2**

## EUROPEAN PATENT APPLICATION

(21) Application number: **92102078.0**

(22) Date of filing: **07.02.92**

(51) Int. Cl.⁵: **C12P 21/00**, //(C12P21/00, C12R1:645)

(30) Priority: **07.02.91 YU 226/91**

(43) Date of publication of application:
**12.08.92 Bulletin 92/33**

(84) Designated Contracting States:
**AT DE IT NL**

(71) Applicant: **KRKA, tovarna zdravil, p.o.**
**Cesta herojev 45**
**YU-68000 Novo Mesto(YU)**

(72) Inventor: **Petkovic, Tomislav**
**Pod Trsko goto 48**
**YU-68000 Novo Mesto(YU)**

(74) Representative: **Patentanwälte Deufel, Hertel, Lewald**
**Postfach 26 02 47 Isartorplatz 6**
**W-8000 München 26(DE)**

(54) **Process for the production of protease C.**

(57) There is disclosed a process for the production of protease C, wherein a mould strain of *Acremonium chrysogenum* species, deposited in VNIIA collection under No. 226A, is cultivated on nutrient substrates containing sources of carbon, of nitrogen, of mineral salts, of vitamins A and D, of B complex vitamins and especially amino acid methionine, under aerobic submerged conditions in the pH range of 5.7 to 8.2 and at a temperature of 26 to 28 °C while aerating and stirring. The isolated protease C is used in industry, agriculture and medicine.

EP 0 498 452 A2

*Technical Field*

The invention belongs to the field of biotechnology and relates to the production of proteolytic enzymes by means of mould strain of *Acremonium chrysogenum* species. Said microorganism is deposited in a collection of microorganisms producing biologically active substances VNIIA, Vsesojuzny nauchno issledovatelsky institut antibiotikov, U.S.S.R, Moscow, under No. 226A.

*Prior Art*

It is well known that proteolytic enzymes are produced by microorganisms, mainly moulds and bacteria. The production of proteolytic enzymes by means of different mould species, species *Streptomyces*, species *Bacillus* and other species of microorganisms is disclosed in several patents.

*Technical Problem*

There was a need for producing an isolate of the *Acremonium chrysogenum* species with high yields of proteolytic enzymes.

*The Technical Solution*

Now it has been unexpectedly found that said isolate of *Acremonium chrysogenum* species, cultivated on appropriate nutrient substrates, aerobically, submerged, in fermentors, under appropriate cultivating parameters, gives high yields of proteolytic enzymes, about 35,000 LV units per ml of fermentation culture filtrate.

Proteolytic enzymes produced by strain producer of *Acremonium chrysogenum* species are: two serine proteinases, I with isoelectric point pI 10.0, II with pI 4.0, molecular weight of said proteinases being 28,000; metaloproteinase with pI 4.6, molecular weight 32,000; carboxypeptidase with pI 5.6, molecular weight 32,000; and aminopeptidase. For practical reasons, the whole complex of said proteinases is referred to as protease C.

Morphological characteristics of the strain producer were established on the basis of morphological forms that appeared if it was cultivated on a substrate of the following composition (sporulation substrate):

| | |
|---|---|
| yeast extract | 4.0 |
| enzymatic hydroliysate of casein | 3.0 |
| glucose (anhydrous) | 10.0 |
| sodium chloride | 1.0 |
| ferrous sulfate | 0.05 |
| manganese sulfate | 0.05 |
| methionine-l.d. | 0.05 |
| agar (bacteriological) | 25.0 |
| distilled water | to 1000 ml |

The pH of substrate thus prepared was corrected with NaOH to 6.7; agar was dissolved, the substrate was poured into appropriate test tubes ad sterilized for 20 minutes at 120 °C. Slant agars were prepared in test tubes of 20mm diameter. Into each test tube 18 ml substrate was poured. Test tubes were arranged into a slant position so that the thickness of agar layer at the bottom of the test tube was 5mm.

Into Petri dishes the substrate with agar was poured in such an amount that the thickness of the layer was 2 or 5 mm; namely, for micellar growth under formation of conidiophores and conidia a thinner layer is necessary, whereas for the formation of arthrospores a thicker substrate layer is necessary.

The strain producer is able to form different cellular forms in connection with arthrospore formation on the one hand or conidia carriers with conidia on the other hand.

We have found that after 12 to 15 days of cultivating on the said substrate (slant agar, Petri dishes) at a temperature of 27 °C, the differentiation of the culture takes place. On thicker agar layers micell fragments and thus arthrospores are formed, while on thinner agar layers conidia carriers are formed on hiphae, which carriers are positioned perpendicularly to vegetative hyphae of air micell. On the top of conidia carriers there is a pseudohead formed by conidiospores, which conidiospores are glued with mucus. The width of the base of conidia carriers is 1.2 $\mu$m. Cross-barriers in a base of conidia carriers were not observed. The length of conidia carriers is 36 $\mu$m and the width from 1.3 to 2 $\mu$m. The dimensions of conidia vary. On a average they amount to 2.4 x 7.9 $\mu$m. Conidia are either biconical or elipsoid in shape.

Individual colonies on agar with layer thickness 5 mm in Petri dishes reach a diameter from 9 to 13 mm. Colonies are cream coloured with a pink shade, umbonate, undulate, with regularly arranged radial convexities.

The producer strain has the following physiological characteristics: optimum growth temperature of 26 to 28 °C; it is a strict aerob; it peptonizes milk in 7 days without coagulating the latter; it hydrolyses starch; and it dissolves gelatine in 3 days.

Beside protease C, it synthesizes two amylases, pectinase, lipase and cellulase. The strain is auxotroph, unable to synthesize amino acids containing sulphur, especially methionine.

It assimilates arabinose, galactose, glucose, lactose, maltose, mannite, raffinose and starch.

It poorly assimilates xylose and ramnose.

It does not assimilate inositol.

The strain producer is genetically stable and it retains morphological as well as physiological characteristics for a practically unlimited number of generations if it is kept on a sporulation substrate in refrigerator at 4 °C.

On the basis of morphological and physiological characteristics it was found that the microorganism producer belongs to genus *Acremonium,* species *Acremonium chrysogenum.*

The inventive process for the production of protease C by means of fermentation is characterized in that the strain producer of *Acremonium chrysogenum* species is cultivated on complex substrates under aerobic submerged conditions.

Nutrient substrates for the production of protease C by means of *Acremonium chrysogenum* species must contain sources of carbon, of nitrogen, of mineral salts, of vitamins A and D, of B complex vitamins and especially amino acid methionine.

Carbon sources are starch, raffinose, sucrose, glucose, ammonium acetate and cod-liver oil.

Mineral salts are contained in calcium carbonate (ground limestone) and in soya meal. The microorganism producer is cultivated in the pH range of 5.7 to 8.2, depending upon the nutrient medium and the stage of development, at a temperature of 26 to 28 °C under appropriate aeration and stirring.

Protease C is isolated in several ways with regard to the use thereof. If the fermentation culture is directly dried in a spray-drier, a "feed grade" quality product is obtained. Pure protease C is obtained from the filtrate of the fermentation culture by ultrafiltration method or precipitation with suitable organic solvents and salts.

Protease C exhibits maximum activity at pH 10.5 - 11.0 but it is also very active in the pH range of 6.0 to 11.0.

The optimum temperature for protease C action is 45 to 55 °C; but it is also very active at a temperature of 25 to 30 °C.

Protease C exhibits an action on proteins of vegetal as well as of animal origin. It disintegrates globular proteins and very effectively scleroproteins collagen, keratin, elastin as well as proteids: glycoproteins, lipoproteins, phosphoproteins, metalloproteins.

It cleaves peptide bonds from 35 to 70%, depending upon the substrate to which it acts.

Hydrolysates obtained contain up to 30% of free amino acids; the amount of peptide varies from 60 to 80% and peptides consist of 2 to 3 amino acid residues.

Protease C is useful in industry, agriculture and medicine. The use of protease C is also one of the objects of the invention.

Formulations of protease C are used:
- in leather industry for removing hair from hide (depilation), softening (bating) of hides and skimming of undesired keratin layers from the hide;
- as the enzymatic component of powdery and liquid washing agents for washing textile products as well as for washing dishes and sanitary installations;
- in dairy industry for coagulation of milk and for preparing some kinds of hard cheese;
- in tobacco industry at the preparation of tobacco for removal of undesired proteins;
- in textile industry after destarching of textile for removal of proteins that were present in starch and remained on textile;
- in brewing industry for preparing fermentation substrates;
- in food industry for decomposition of complex, harder-digestible proteins (soya beans, peanuts, meat etc.) into simpler, easily digestible components: lower proteins, polypeptides, peptides and amino acids;
- in slaughter houses for decomposition of proteins of blood, of sinews, of ligaments, of fasciae, of hair, of feathering etc. into digestible lower proteins, polypeptides, peptides and amino acids;

- for purification of waste water containing proteins so that said proteins are decomposed into polypeptides, peptides and amino acids, which are further decomposed by microflora of waste water into carbon dioxide and ammonia;
- in combination with cellulase and pectinase for preparing ensilage of higher quality, i.e. ensilage not containing harder-digestible higher proteins but easily digestible lower proteins, polypeptides, peptides and amino acids;
- in breeding calves, pigs, chicken and fish certain amounts of a protease C formulation are added into fodder, whereby the digestion of protein components of the fodder improves, which results in a quicker growth and weight increase of fattened animals on the one hand and in a smaller consumption and better fodder utilization on the other hand;
- in combination with a broad spectrum antibioticum in the form of cream in human medicine and in veterinary medicine for treatment of burns, chronical ulcerous wounds, panaritia of ungulates and generally infected wounds, where also necrotic tissue appears. Protease C decomposes necrotic tissue; the antibioticum suppresses infection; quick and regular granulation of wounds as well as regeneration of injured tissues take place.

Those skilled in the art can determine the appropriate amount and the manner how to use protease C formulation by testing.

The invention is illustrated by the following Examples:

*Example 1*

Production of microorganism producer for fermentation and biosynthesis of protease C

Microorganism producer multiplied and was kept on slant sporulation agars. Slant agars were inoculated with a suspension of either arthrospores or conidiospores and were incubated in a thermostat at the temperature of 27 ° C. After incubation for 48 to 72 hours, the surface of slant agar was overgrown with micellar structures. There followed the differentiation of hyphae: on thicker agar layers with fragmentation into arthrospores, whereas on thinner agar layers, on aerobic hyphae, there grew conidia carriers with conidiospores. Complete sporulation of agar took place after incubation for 12 to 15 days. Agars thus prepared were deposited in a refrigerator at a temperature of 4 to 8 ° C and served to keep or further multiply the strain producer. The durability of agars thus prepared was at least one year.

Sorghum substrate was inoculated with a suspension of spores from fresh, 12 to 15 days old slant agar, rich with arthrospores.

Sorghum (Sorghum bicolor) of hybar sort or "zrnaš" sort was used.

Into Erlenmeyer flasks of 1000 ml, each time 300 g of sorghum were given and 100 ml of tap water were added. The flasks were closed with a cotton wool stopper. The stoppers were covered with paper and the paper was fixed to the flask neck with a rubber ring.

The substrate was sterilized twice in a 24 hour interval at the temperature of 120 ° C for 45 minutes. Between the sterilizations, the flasks containing sorghum were thermostated at the temperature of 35 ° C for germinating possibly surviving spores residing on sorghum, and these germs were destroyed in the second sterilization. Arthrospores from one slant agar were suspended in 100 ml sterile tap water. Each flask containing sorghum was inoculated with 10 ml of the suspension of spores. Thereafter flasks containing sorghum were thoroughly shaken so that the inoculum was uniformly arranged and the sorghum was loosened and thus the admission of air was made possible.

The inoculated flasks containing sorghum substrate were incubated for 12 to 15 days in a thermostat at the temperature of 27 ° C. During this time sorghum grains overgrew the micell, which fragmented into arthrospores, and the culture thus prepared was used for tests and for production.

The cultures on sorghum were deposited in a refrigerator at a temperature between 4 ° C and 8 ° C. They were taken from the refrigerator when necessary. The durability of these cultures, if prepared and deposited in the above manner, was at least one year.

*Example 2*

Production of culture of microorganism producer in propagator

| Composition of propagator substrate (%): | |
|---|---|
| maize extract | 2.0 |
| sucrose | 2.0 |
| calcium carbonate (Contraplex) | 2.0 |
| ammonium acetate | 0.4 |
| cod-liver oil | 1.4 |
| tap water | to 100.0 |

The propagator was filled to two thirds. The substrate was sterilized for 45 minutes at the temperature of 120 °C and cooled to 27 °C. The pH of substrate after sterilization was 6.0.

The propagator substrate was inoculated with the culture on sorghum, prepared in such a way as disclosed in Example 1. The amount of inoculum was 0.4% v/v.

The cultivating parameters were a temperature of 26 to 28 °C and an aeration of 0.5 l/l of substrate per minute. During the cultivation of the propagator culture the concentration of the dissolved oxygen in culture should not drop under 30% with respect to the concentration of the dissolved oxygen at the beginning of the propagator culture cultivation. With this in view, the type and the size of stirring elements and the number of revolutions of the stirrer were determined. For small vessels of 400 ml, the necessary number of revolutions of the stirrer was 400 per minute. The stirring elements were turbines, whose diameters amounted to 1/3 diameter of the vessel.

The characteristics of a ripe propagator culture with which the pre-fermentor was inoculated were as follows: age 40 to 48 hours; pH 7.2 to 7.4; microscopic picture: lush micellar growth, the culture contained 10 to 20% of micell (centrifugation in cuvettes, 3000 revolutions, duration 10 minutes).

*Example 3*

Production of culture of microorganism producer in pre-fermentor

The composition and the preparation of the pre-fermentor substrate were the same as in the propagator substrate described in Example 2. The pre-fermentor was inoculated with 10% of propagator culture v/v, prepared as in Example 2. The cultivation parameters were the same as in the propagator cultivation step. The difference lay in the number of revolutions of the stirrer. For vessels of 4 $m^3$ volume 180 rpm was necessary. The stirring elements were turbines, whose diameter amounted to 1/3 diameter of the vessel (pre-fermentor).

The characteristics of the ripe pre-fermentor culture were as follows: age 24 to 30 hours; pH 7.0 to 7.2; microscopic picture: lush micellar growth; the culture contained 12 to 16% of micell (centrifugation in cuvettes, 3000 revolutions, duration 10 minutes).

*Example 4*

Protease C biosynthesis in fermentor

Characteristics of equipment and fermentor:

Material: stainless steel.
Height/width ratio of fermentor: 2.5 : 1.
Electromotor of 4 kW per $m^3$ of fermentation culture.
Appropriate means for breaking the flow of fermentation culture. Stirrer having three stirring elements of open turbine type; the diameter of stirring elements amounts to 1/3 of fermentor diameter.
Sparger, positioned over the fermentor bottom, having a somewhat smaller diameter than the diameter of stirring elements, with appropriate openings in the lower side.
Apparatus for controlling the air flow-rotameter.
System for temperature maintaining and controlling.
Manometer for pressure controlling in the fermentor.
System for pH controlling.
System for controlling the amount of dissolved oxygen in fermentation culture.
System for controlling and suppressing foam in fermentor.

EP 0 498 452 A2

| Composition of fermentor substrate (%): | |
|---|---|
| starch | 6.0 |
| soya meal | 7.3 |
| dry yeast | 0.5 |
| calcium carbonate (Contraplex) | 1.0 |
| l,d-methionine | 1.0 |
| cod-liver oil | 4.0 |
| tap water | to 100.0 |

The substrate was prepared either directly in the fermentor so that one half of the total water amount was added into the fermentor and then step by step the substrate components and water to intended volume were added while stirring, or the substrate components were dissolved, suspended and emulgated in the appropriate amounts of water in a stirrer and were trasported *via* pipes into the fermentor. Finally water was added to the intended volume of 67%.

The substrate was sterilized for 45 minutes at the temperature of 120 °C and cooled to 27 °C. The pH of the substrate after sterilization was between 6.6 and 6.9. The substrate thus prepared and sterilized was inoculated with 10% of pre-fermentor culture v/v, prepared as in Example 3. Thus the final volume of the fermentation culture amounted to 73% of the absolute fermentor volume.

Cultivating parameters

The critical point in the biosynthesis of protease C was the concentration of the dissolved oxygen in the fermentation culture. Under the pressure of the fermentation culture column, the air flow originating from sparger openings broke into bubbles, which were received by stirring elements and the latter disperged them into still smaller bubbles, with the result that in contacting great surfaces of air bubbles and fermentation culture a good dissolution of oxygen was made possible.

The rate of protease C synthesis and the amount thereof depended upon the amount of dissolved oxygen in the culture.

The concentration of dissolved oxygen in the fermentation culture should not fall under 10% with regard to the oxygen concentration at the beginning of the cultivation referred to as 100%.

The stirring on the one hand resulted in oxygen dissolution and on the other hand regulated the amount of the micell in the fermentation culture. Insufficient strirring and an insufficient amount of the dissolved oxygen in the fermentation culture caused an enormous development of the micell. The culture also contained up to 90% of the micell (centrifugation in cuvettes, 3000 revolutions, 10 minutes), which resulted in a drop of the dissolved oxygen concentration to zero and in the suppression of the culture. At appropriate stirring the oxygen amount never dropped under 10% and the amount of the micell in the culture never exceeded 55 to 60%, which was the optimum for protease C biosynthesis.

We have found out, that in the first cultivating step, from 0 to 90 hours, the stirring with 150 rpm is necessary, wherefor a motor power of 4 kW per $m^3$ of the fermentation culture is sufficient for the disclosed fermentor type and stirring elements. In the second cultivation step, from 90 to 120 hours, the stirring with 130 rpm and a motor power of 2.5 kW per $m^3$ of the fermentation culture is sufficient.

For the aeration of the fermentation culture the air amount 0.5 l/l of culture per minute is sufficient. The air amount of 0.8 to 1.0 l/l of culture per minute is more suitable. Although a greater air amount does not enhance the amount of dissolved oxygen in the culture, the culture is raised, its density is reduced and thus the motor load is reduced.

The development of microorganism producer in cultivation is controlled. Native microscopic preparations are used to that effect. At the conditions, which are the optimum for protease C biosynthesis, the vegetative micell added from the pre-fermentor is differentiated in the fermentation culture in arthrospores, which grow individually in pairs and irregular conglomerates. In the next step, from arthrospores hyphae forming micellar structures grow again. The process of transformation of micell into arthrospores and *vice versa* is repeated periodically. In this way the biomass in the culture increases to the concentration, which is the optimum for protease C synthesis.

The culture was cultivated at 27 °C ± 1 ° in the whole process of protease C biosynthesis.

At cultivation an overpressure of 0.3 bar was maintained in the fermentor.

The pH of the culture dropped from the starting pH 6.6 - 6.9 to 6.1 - 5.9 after 40 to 45 hours, then it increased to 6.5 after 65 hours of cultivation. The pH of 6.5 was the optimum for protease C biosynthesis and was maintained almost to the end of the fermentation. The increase of pH to 7.0 - 8.2 after 110 - 120

6

hours of fermentation indicated the end of the fermentation.

During the cultivation the fermentation culture increased its volume for 1/5 with regard to the volume of the charged substrate due to the increased viscosity of the culture and the resulting accumulation of air bubbles and carbon dioxide formed in the culture. At the end of the fermentation, foam appeared on the culture surface, which foam was suppressed with silicone 1510, produced by Dow Corning.

The protease C biosynthesis was completed after 110 - 120 hours of cultivating at the disclosed conditions. It was evidenced by the foaming of the culture, by the pH increase to 7.0-8.2, and by the stopping of the increase of enzymatic activity in the culture.

The enzymatic activity at the end of the cultivation was 22,000 to 24,000 LV units/ml of the culture filtrate.

### Example 5

Protease C biosynthesis was carried out in such a way and under such conditions as disclosed in Example 4 with the difference that during cultivating the culture was additionaly fed, i.e. after cultivating for 72 hours 1% of maize starch and 1% of cod-liver oil were added and after 96 and 120 hours 1% of cod-liver oil with regard to the charged substrate volume were added to the culture. In this way the culture cultivation was prolonged to 150 hours and an enzymatic activity of protease C of 35,000 LV units/ml of the culture filtrate was achieved.

Protease C preparations and their production from the fermentation culture of *Acremonium chrysogenum*

Protease C was isolated in several ways according to the application thereof.

### Example 6

By direct drying of the fermentation culture in a spray drier a preparation of "feed grade" quality was obtained.

At the completion of cultivating, 1000 l of fermentation culture of *Acremonium chrysogenum* having the activity of 35,000 LV units/ml of the culture filtrate were dried directly in a spray drier at an input temperature of 200-220 °C and an output temperature of 80-85 °C.

About 100 kg of a "feed grade" quality enzymatic preparation in a cream coloured fine powdery form with a 5% humidity and an activity of 300,000 LV units/g of the preparation were obtained. The pH of 1% suspension of the preparation was 7.0 to 7.2. The yield as to dry substance in the fermentation culture was 90% and as to enzymatic activity 85%.

### Example 7

1000 l of the fermentation culture without any additives were filtered on a vacuum rotation filter. A layer of a filter agent (decalite, randalite) had been previously applied to the filter cloth. After the filtration the filter cake was washed with so much deionized water that the end volume of the filtrate was about 1000 l.

Ultrafiltration was carried out through a membrane up to maximum permeability of solubles with molecular mass 20,000. By ultrafiltration the starting volume of the filtrate was concentrated to 1/3 volume. The obtained concentrate was diluted with deionized water to the starting volume of the filtrate and said operation was repeated three times.

By the above ultrafiltration process, most undesired solubles having molecular mass up to 20,000 were removed.

The concentrate thus prepared was dried in a spray drier. 40 kg of technical enzymatic preparation in a greyish cream coloured fine powdery form with a 5% humidity and an activity of 600,000 LV units/g of the preparation were obtained. The pH of 1% suspension of the preparation was 7.0 to 7.2. The yield as to enzymatic activity in the fermentation culture was 70%.

### Example 8

The enzymatic concentrate obtained by ultrafiltration according to Example 7 was treated with isopropanol in an isopropanol:concentrate ratio 1:1. Thereby protease C precipitated. The obtained precipitate was separated from the liquid phase by filtration on a shoulder filter press. The filter cake on the filter press was blown through with air to separate as much liquid phase as possible. The cake was skimmed from the filter press cloth and dissolved in 5 mM acetate buffer with a pH of 5.0. Calcium acetate was used for preparing the buffer. The acetate buffer was added step by step in the amount necessary for dissolving the enzymatic cake under intense stirring. According to said process the yield was 60% as to the enzymatic activity in the fermentation culture. The enzymatic concentrate in acetate buffer was treated with 6% of active carbon. Active carbon bound impurities and pigments. There followed the filtration on a shoulder filter press. In this way a purified and non-coloured enzymatic concentrate in acetate buffer was obtained.

When the concentrate was dried in a spray drier, 15 kg of pure quality enzymatic preparation in a white fine powdery form with 5% humidity and an activity of 1,200,000 LV units/g of the preparation were obtained. The pH of 1% suspension was 7.0 to 7.2. After this operation the yield was 50% as to the enzymatic activity in the fermentation culture.

*Example 9*

Non-coloured enzymatic concentrate in acetate buffer was salted out with ammonium sulfate. Therefor it was necessary to achieve an 80% concentration of saturation of ammonium sulfate in said concentrate.

The obtained precipitate was filtered off and dissolved in 0.1 M acetate buffer, pH 5.0. There followed a dialysis through a cellophane membrane.

The enzymatic solution in 0.1 M acetate buffer was poured into cellophane bags and then the bags were immersed into 5 mM buffer solution, pH 5. The dialysis process was repeated three times in 12 hours.

After dialysis the enzymatic concentrate was dried in a lyophilisation drier. A very pure protease C preparation in the amount of 5 kg with an activity of 2,500,000 LV units/g of preparation was obtained. The yield as to enzymatic activity in the fermentation culture was 35%.

*Analysis of protease C proteolytic activity*

An analysis of protease C proteolytic activity either in the fermentation culture or in dry preparations was carried out according to the Löhlein-Vollhardt method, which was accepted as Yugoslavian standard JUS H.M2.101.

As a proteolytic unit according to Löhlein-Vollhardt, abbreviated p.u. LV, there is understood the activity of enzymatic preparation, which is under testing conditions according to the Löhlein-Vollhardt method able to decompose 1.725 mg of alkaline casein Hamarsten (Merck). The amount of proteolytic units in 1 g of preparation is accepted as the proteolytic value of that preparation.

*Use Example 1*

Use of protease C preparation in leather industry

Treatment of cowhides
Raw material: raw cowhides
Auxiliary materials:
Nonionik AP-6
$Na_2S$
$Ca(OH)_2$
HCl
$NH_4Cl$
lactic acid
Dekaltat N (for decalcifying)
protease C preparation (activity 2,500 LV units/g)
NaCl
$H_2SO_4$
formic acid
basic chromosulfate
Kutrilix WWL
NaOH (soda)
calcium formate
$NaHCO_3$
Tokanol
Chromsulfol K

Procedure

Pre-soaking for 60 minutes with 200% of water at 25 °C, 0.2% of Nonionik AP-6, followed by washing with water. Wetting and liming for 2 hours with 200% of water at 28-30 °C, 0.3% of protease C preparation, then adding 2-3% of $Na_2S$, duration 1.5 hours, then adding 3% of lime, duration 16 hours, under periodical stirring. After said operations hides should be thoroughly washed with running water. There followed the machine removal of subcutaneous tissue, manual trimming, weighing and washing of the hides with running water.

Decalcifying and bating was carried out with 150% of water at 30 °C, 0.2% of hydrochloric acid, 0.1% of ammonium chloride, 0.3% of Dekaltat N, 0.2% of lactic acid. The treatment was carried out for 30 to 45 minutes at pH 8.5. Then 0.2% of the protease C preparation were added and it was treated for 20 to 30 minutes. The success of bating was controlled by bladder and finger. There followed the washing with running water.

Pickling was carried out for 10 minutes with 70% of water at 28-30 °C, 8% of sodium chloride, then 0.4% of sulfuric acid and 0.8% of formic acid were added and it was treated for 2 hours at pH 3.6.

Tanning: there were added 7.5% of basic chromosulfate in two portions, each time one half of the total amount. It was treated for 30 minutes each time. 1% of Kutrilix WWL was added, it was treated for 16 hours and then the pH was adjusted with NaOH to 3.6-3.8. After tanning the hides were let to stay for 2 days, dried and thinned to a thickness of 1.4 mm, neutralized with calcium formate and calcium bicarbonate. Then they were fetted with Tokanol, Kutrilix WWL and Chromsulfol K. After fetting the hides were dried and formed. The obtained hide was full, of fine clean grain, smooth to the touch and could be used for different articles.

Similarly as cow hides, calf hides, sheep hides, goat hides, pig hides and hides of other domestic animals as well as of game were prepared.

### Use example 2

Use of protease C in washing agents

Therefor a technical protease C preparation with an activity of 6,000,000 LV units/g of preparation was used. From this preparation enzymatic granules with an activity of 150,000 LV units/g of granules were prepared.

Procedure for preparing granules

As protease C carrier, pentasodium triphosphate or Na citrate (both being otherwise components of washing agents) was used as well as titanium dioxide, which gives white colour to the granules and equalizes them with the other components of the washing agent as to colour.

From said components, i.e. protease C, the carrier and $TiO_2$, a homogenous powdery blend was prepared, the powder particle size being about 500 $\mu$m. The blend contained 1.5% of $TiO_2$, 30% of the protease C preparation and 68.5% of the carrier.

An aqueous solution containing 5% of PVP (polyvinyl pyrrolidone) K-30 and 0.5% of Tween 80, in an amount of 21% as to the powdery blend was prepared.

Said powdery blend and the aqueous solution were successively injected into the granulating system of the granulating apparatus, where at a temperature of 40 °C granules were formed. The granule dimensions were determined according to the wishes of the customer and according to the dimensions of the granules of other components of the washing powder.

Into the washing powder 2 to 4% of enzymatic granules containing 150,000 LV units of protease C per g of granules were added.

### Claims

1. Process for the production of protease C, characterized in that a mould strain of *Acremonium chrysogenum* species, deposited in VNIIA collection under No. 226A, is cultivated on nutrient substrates containing sources of carbon, of nitrogen, of mineral salts, of vitamins A and D, of B complex vitamins and especially amino acid methionine, under aerobic submerged conditions in the pH range of 5.7 to 8.2 at a temperature of 26 to 28 °C while aerating and stirring, whereby the concentration of the dissolved oxygen in the fermentation culture should not fall under 10% with regard to the oxygen concentration at the beginning of the cultivation, and then isolated.

2. Process according to claim 1, characterized in that the nutrient substrate preferably contains cod-liver oil in combination with maize starch as the carbon source, soya meal, dry yeast and methionine as the nitrogen source, and calcium carbonate as the mineral source.

3. Use of protease C in industry, agriculture and medicine.

4. Use of protease C according to claim 2 in leather industry for removing hair from hide (depilation), softening (bating) of hides and skimming of undesired keratin layers from the hide; as the enzymatic component of powdery and liquid washing agents for washing textile products as well as for washing dishes and sanitary installations; in dairy industry for coagulation of milk and for preparing some kinds of hard cheese; in tobacco industry at the preparation of tobacco for removal of undesired proteins; in textile industry after destarching of textile for removal of proteins that were present in starch and remained on textile; in brewing industry for preparing fermentation substrates; in food industry for decomposition of complex, harder-digestible proteins (soya beans, peanuts, meat etc.) into simpler, easily digestible components: lower proteins, polypeptides, peptides and amino acids; in slaughterhouses for decomposition of proteins of blood, of sinews, of ligaments, of fasciae, of hair, of feathering etc. into digestible lower proteins, polypeptides, peptides and amino acids; for purification of waste water containing proteins so that said proteins are decomposed into polypeptides, peptides and amino acids; in combination with cellulase and pectinase for preparing ensilage of higher quality, i.e. ensilage not containing harder-digestible higher proteins but easily digestible lower proteins, polypeptides, peptides and amino acids; in breeding calves, pigs, chicken and fish; in combination with a broad spectrum antibioticum in the form of cream in human medicine and in veterinary medicine for treatment of burns, chronical ulcerous wounds, panaritia of ungulates and generally infected wounds, where also necrotic tissue appears.